(19) Europäisches Patentamt
European Patent Office
Office européen des brevets

(11) Publication number: 0 117 578 A2

# (12) EUROPEAN PATENT APPLICATION

(21) Application number: 84200181.0

(22) Date of filing: 08.02.84

(51) Int. Cl.³: **C 07 D 233/60**, C 07 D 249/08, C 07 D 401/14, C 07 D 405/14, C 07 D 403/06, C 07 D 405/06, A 01 N 43/50, A 01 N 43/64, A 61 K 31/41, A 61 K 31/415 // C07D307/32

(30) Priority: **23.02.83 JP 30023/83**
**14.03.83 JP 42728/83**
**25.04.83 JP 73558/83**

(43) Date of publication of application: **05.09.84 Bulletin 84/36**

(84) Designated Contracting States: **BE CH DE FR IT LI NL SE**

(71) Applicant: **SHIONOGI & CO., LTD., 12, Dosho-machi 3-chome Higashi-ku, Osaka 541 (JP)**

(72) Inventor: **Ogata, Masaru, 8-20-8, Sumiyoshiyamate Higashinada-ku, Kobe-shi Hyogo (JP)**
Inventor: **Matsumoto, Hiroshi, 5-10-25, Yamatedai, Ibaraki-shi Osaka (JP)**
Inventor: **Tawara, Katsuya, 1-1-814, Higashiota, Ibaraki-shi Osaka (JP)**
Inventor: **Ishiguro, Takeo, 2-16-43, Kusatsu, Kusatsu-shi Shiga (JP)**
Inventor: **Hatta, Takayuki, 116-38, Sagami Koka-cho, Koka-gun Shiga (JP)**

(74) Representative: **Bruin, Cornelis Willem et al, OCTROOIBUREAU ARNOLD & SIEDSMA Sweelinckplein 1, NL-2517 GK The Hague (NL)**

(54) **Azole-substituted alcohol derivatives.**

(57) A compound of the formula:

$$R^2\text{–}A\text{–}\underset{R^3}{\overset{OR^1}{\overset{|}{\underset{|}{C}}}}\text{–}CH_2\text{–}Q$$

(wherein
A is C=O, CH(OH) or C($C_2$–$C_5$ alkyl)(OH),
Q is imidazolyl or triazolyl,
$R^1$ is hydrogen, $C_1$–$C_5$ alkyl, or $C_1$–$C_8$ acyl, and
$R^2$ and $R^3$ each is $C_1$–$C_5$ alkyl, $C_3$–$C_6$ cycloalkyl, $C_2$–$C_6$ alkenyl, benzyl optionally substituted by 1 to 3 halogens, 5- or 6-membered heterocycle containing N, O or S or phenyl optionally substituted by 1 to 3 substituents selected from $C_1$–$C_3$ alkyl, $C_1$–$C_3$ alkoxy and halogen) or its acid addition salt, being useful as oral antimycotics or agricultural fungicides, is provided from several routes.

EP 0 117 578 A2

0117578

The present invention relates to azole-substituted alcohol derivatives. More particularly the invention is directed to a compound of the formula:

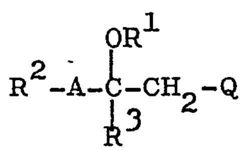

(I)

(wherein A is C=O, CH(OH) or C($C_1$-$C_5$ alkyl)(OH),

Q is imidazolyl or triazolyl,

$R^1$ is hydrogen, $C_1$-$C_5$ alkyl or $C_1$-$C_8$ acyl, and

$R^2$ and $R^3$ each is $C_1$-$C_5$ alkyl, $C_3$-$C_6$ cycloalkyl, $C_2$-$C_6$ alkenyl, benzyl optionally substituted by 1 to 3 halogens, 5- or 6-membered heterocycle containing N, O or S or phenyl optionally substituted by 1 to 3 substituents selected from $C_1$-$C_3$ alkyl, $C_1$-$C_3$ alkoxy and halogen)

or its acid addition salts, being useful as oral antimycotics or agricultural fungicides.

In the definition of the formula (I) $C_1$-$C_5$ alkyl refers to methyl, ethyl, propyl, isopropyl, butyl, isobutyl, sec-butyl, t-butyl pentyl, isopentyl or sec-pentyl; $C_1$-$C_3$ alkoxy refers to methoxy, ethoxy or propoxy; $C_2$-$C_6$ alkenyl refers to vinyl, 1-propenyl, 2-propenyl, 1-butenyl, 2-butenyl, 3-butenyl, 2-pentenyl or 3-hexenyl; $C_3$-$C_6$ cycloalkyl refers to cyclopropyl, cyclobutyl, cyclopentyl or cyclohexyl; $C_1$-$C_8$ acyl refers to formyl,

acetyl, propionyl, butynyl, valeryl, benzoyl, phenylacetyl, methoxycarbonyl or ethoxycarbonyl; 5- or 6-membered heterocycle containing N, O, or S refers to pyridyl, furyl or thienyl; halogen refers to fluorine, chlorine, bromine or iodine; and triazolyl refers to 1H-1,2,4-triazol-1-yl or 4H-1,2,4-triazol-1-yl.

Ketoconazole is commercially available as an oral antimycotic in Europe and U. S. A. but has a disadvantage of adverse reactions such as hepatic dysfunction (U. S. pats. 4,144,346 and 4,223,036). Further European Unexamd. Pat. Publn. No. 61835 discloses azole-substituted propanols but their utility is directed only to agricultural fungicides. It has been discovered by the present inventors that the novel azole-substituted alcohol derivatives (I) exhibit orally active potent antimycotic activity as well as agriculturally fungicidal activity. Thus the present invention has been established by these findings.

The compounds (I) of the present invention can be prepared through several synthetic routes as shown below.

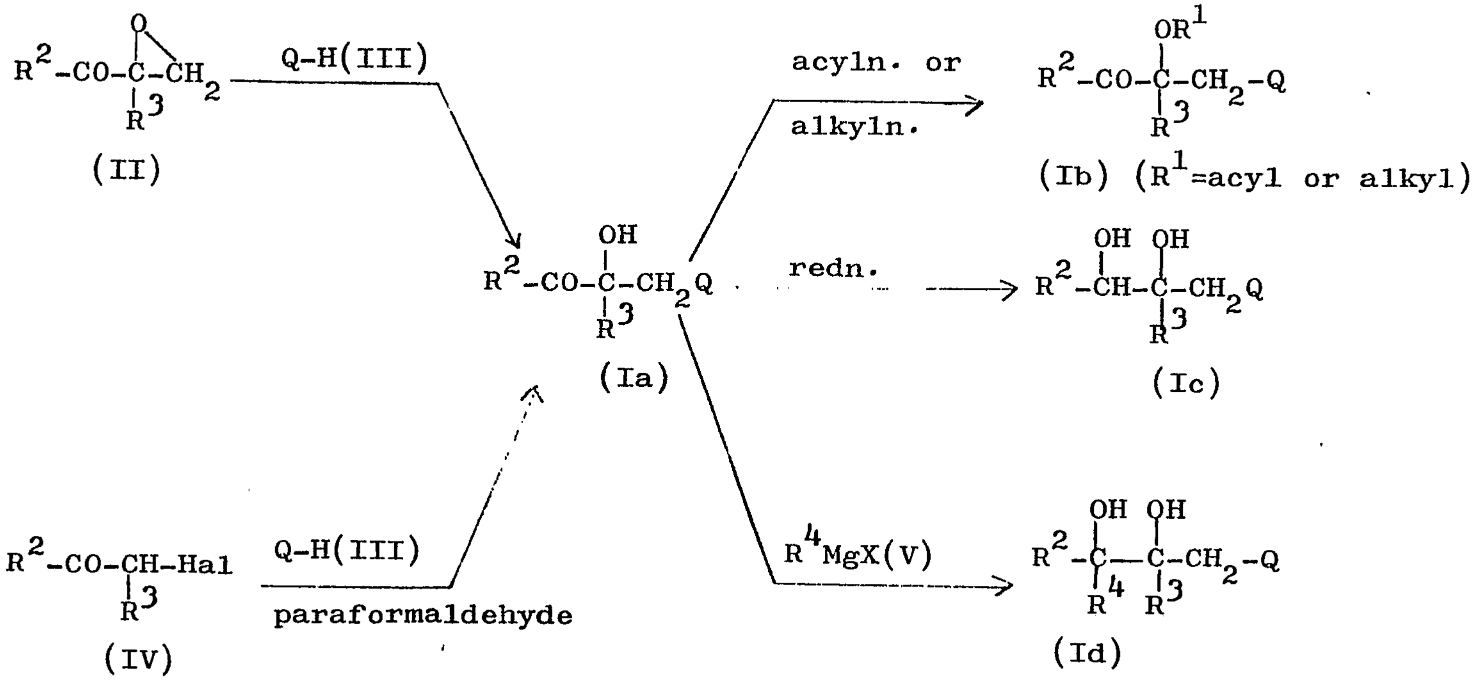


(II) (IV) (Ia) (Ib) ($R^1$=acyl or alkyl) (Ic) (Id)

(wherein Hal and X each is halogen, $R^4$ is $C_1$-$C_5$ alkyl, and Q, $R^2$ and $R^3$ each is as defined above)

According to the process provided by the present invention, the azole-substituted alcohol derivatives (I) or their acid addition salts are manufactured by

a) reacting a compound of the formula:

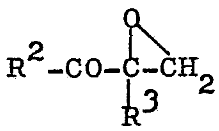 (II)

(wherein $R^2$ and $R^3$ each is as given earlier) with a compound of the formula:

Q-H (III)

(wherein Q is as given earlier) in the presence of an inorganic base in a solvent at temperature of from 20 to 150°C or

b) reacting a compound of the formula:

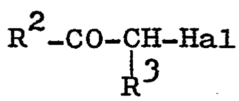 (IV)

(wherein Hal is halogen and $R^2$ and $R^3$ each is as given earlier)

with paraformaldehyde and a compound of the formula:

Q-H (III)

(wherein Q is as given earlier) in the presence of an inorganic base in a solvent at temperature of from 0 to 100°C optionally followed by subjecting the resulting compound of the formula:

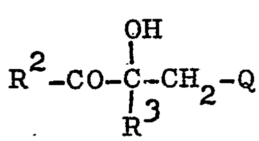 (Ia)

(wherein Q, $R^2$, and $R^3$ each is as given earlier) to (c) acylation, (d) alkylation, (e) reduction or (e) Grignard reaction with a compound of the formula:

$R^4MgX$ (V)

(wherein $R^4$ and X each is as given earlier) in accordance with procedures already known.

Accordingly the ketol (Ia) is prepared by reacting the oxirane (II) with an azole (III) such as 1H-imidazole or 1H-triazole in the presence of an inorganic base such as an alkali metal or alkaline earth metal alkoxide, hydride or hydroxide in a solvent such as hexamethylphosphoric triamide, dimethylformamide, dimethylsulfoxide, tetrahydrofuran, dioxane, methanol, ethanol or mixture thereof at temperature of from 20 to 150°C. In case of using 1H-triazole as Q-H (III), eventually a 4H-1,2,4-triazol-4-yl isomer (Ia-) may be produced together with 1H-1,2,4-triazol-1-yl compound (Ia) but can be separated from the latter in accordance with methods already known, if necessary.

A starting oxirane (II) is prepared in accordance with the following scheme:

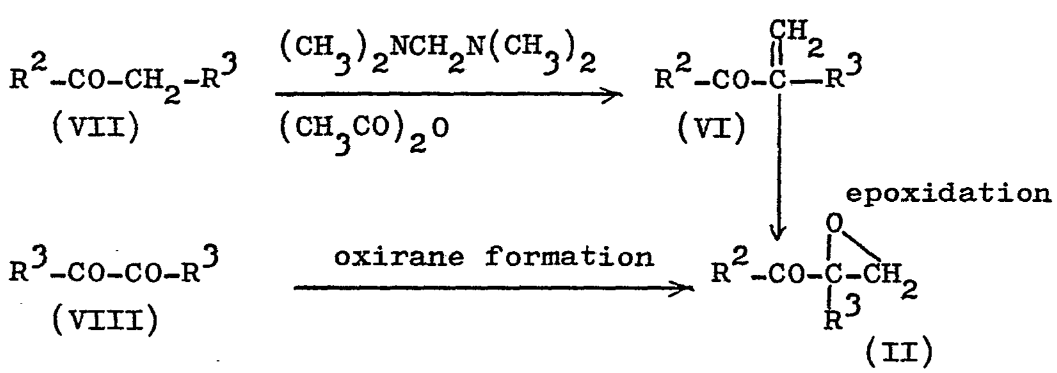


(VII) (VI) (VIII) (II)

Thus the active methylene compound (VII) is allowed to react with bis(dimethylamino)methane in acetic anhydride at temperature of from 0°C to 120°C to give the vinylketone (VI) [Kametani et al., Heterocycles, 12, 933 (1979)], which is then oxidized with a peroxide such as hydrogen peroxide or a peracid such as performic acid, peracetic acid, pertrichloroacetic acid perbenzoic acid, per-m-chlorobenzoic acid or perphthalic acid in a solvent such as a ketone (e.g. acetone, methyl ethyl ketone), an ether (e.g. ether, tetrahydrofuran, dimethoxyethane), a halogenated hydrocarbon (e.g. chloroform, carbon tetrachloride, methylene chloride, 1,2-dichloroethane), an aprotic solvent (e.g. benzene, toluene) or a lower carboxylic acid (e.g. formic acid, acetic acid) at temperature of from -10°C to 60°C.

The oxirane (II) is also prepared by subjecting the diketone (VIII) to the oxirane formation reaction, Thus the diketone (VIII) is allowed to react with diazomethane in a solvent such as dimethylformamide [Eistert et al., Ber. 91, 2710 (1958)] or with dimethyloxosulfonium or dimethylsulfonium methylide in the presence of sodium hydroxide in a solvent such as dimethylsulfoxide [Corey et al., J. Am. Chem. Soc., 87, (6) 1353 (1965)].

In an alternative method, the objective ketol (Ia) is also prepared by reacting a haloketone (IV) with paraformaldehyde and an azole (III) in the presence of an inorganic

base in a solvent such as dimethylsulfoxide, dimethylformamide, hexamethylphosphoric triamide or the like at temperature of from 0 to 100°C. The starting haloketone (IV) is prepared by halogenating a corresponding ketone or ketol (IX) with an appropriate halogenating reagent such as sulfuryl chloride or thionyl chloride with or without solvent (e.g. chloroform, carbon tetrachloride) at temperature of from 10 to 120°C.

$$R^2\text{-CO-}\underset{}{\overset{Y}{\overset{|}{C}}}H\text{-}R^3 \quad (IX) \longrightarrow R^2\text{-CO-}\overset{Hal}{\overset{|}{C}}H\text{-}R^3 \quad (IV)$$

(wherein Y is hydrogen or hydroxy and Hal, $R^2$ and $R^3$ each is as given earlier)

In an optional process, the ketol (Ia) thus obtained is then subjected to acylation, alkylation, reduction or Grignard reaction with the compound of the formula:

$R^4MgX$ (V)

(wherein $R^4$ and X each is as given earlier)

in accordance with procedures already known. Thus the acylation is performed using an acylating reagent such as an anhydride or halide of carboxylic acid in the presence of a base such as organic base (e.g. pyridine, 4-dimethylaminopyridine, triethylamine) or inorganic base (e.g. alkali metal hydride, alkali metal alkoxide, alkali metal hydroxide) with or without solvent (e.g. methylene chloride,

chloroform, dimethylformamide, dimethylsulfoxide) at temperature of from 0 to 100°C. Exemplary acylating reagents are acetic anhydride, methyl chlorocarbonate, propionyl chloride, trifluoroacetyl chloride, benzoyl chloride and p-chlorobenzoyl chloride.

The alkylation of the ketol (Ia) is performed using an alkyl halide such as methyl chloride, ethyl bromide, propyl iodide or butyl bromide or dimethyl sulfate in the presence of a base as afore mentioned in a solvent such as dimethylsulfoxide, dimethylformamide, benzene, hexamethylphosphoric triamide or methylene chloride at temperature of from 10 to 120°C.

The reduction of the ketol (Ia) is performed with an alkali metal complex such as sodium borohydride, potassium borohydride or lithium aluminum hydride. This reaction is generally achieved at temperature of from 15 to 120°C in a solvent such as methanol, ethanol, dimethylformamide, dimethylsulfoxide or the like.

The Grignard reaction of the ketol (Ia) is performed with a compound of the formula:

$R^4MgX$ (V)

(wherein $R^4$ and X each is as given earlier) in a solvent such as ether, dioxane or tetrahydrofuran at temperature of from 0 to 120°C.

The objective compounds (I: Ia, Ib, Ic and Id) have

one or two asymmetric carbon atoms and include optical isomers or diasteroisomers. Though they may be generally obtained in the form of a racemate, it can be resolved into its optical isomers or diastereoisomers in accordance with known methods; for example, with the aid of an appropriate optically active acid or according to known methods such as fractional crystallization of the diastereoisomeric salts obtained.

The compounds (I) of this invention form acid addition salts with an inorganic acid such as hydrohalogenic acid (e.g. hydrochloric acid, hydrobromic acid), sulfuric acid, phosphoric acid or nitric acid and with an organic acid such as acetic acid, succinic acid, glycolic acid, lactic acid, gluconic acid, tartanic acid, citric acid, maleic acid, malic acid, fumaric acid, methanesulfonic acid, p-toluenesulfonic acid, oxalic acid, ascorbic acid, benzoic acid or the like. The pharmaceutically acceptable acid addition salts are preferred in the use of oral antimycotics.

The compounds (I) and their pharmaceutically acceptable acid addition salts possess potent orally active antimycotic activity and may be used as medicaments; for example, in the form of pharmaceutical preparations which contain them in association with one or more of pharmaceutically acceptable carriers, diluents and excipients. Exemplary carriers, diluents and excipients can be organic or inorganic materials

suitable for enteral or parenteral administration (e.g. water, lactose, gelatin, starches, magnesium stearate, talc, vegetable oils, gums, polyalkylene glycols, petroleum jelly, etc.). The pharmaceutical preparations can be made up in a solid form such as tablets, dragees, capsules, pills, granules, powders or suppositories or in a liquid form such as solutions, suspensions or emulsions. The pharmaceutical preparations may be subjected to conventional pharmaceutical operations such as sterilization and/or may contain conventional pharmaceutical adjuvants such as preservatives, stabilizers, wetting agents, emulsifiers, buffers, etc. The dosages in which the compounds (I) and their pharmaceutically acceptable acid addition salts may be admistered can vary depending upon the requirements of the patient and directions of the attending physician. A daily dosage of said compound to a human adult in oral administration is 15 to 2,000 mg and may be administered once or in several divisions.

Presently preferred and practical embodiments of the present invention are illustratively shown in the following Examples, Esperiments and Formulations

Example 1

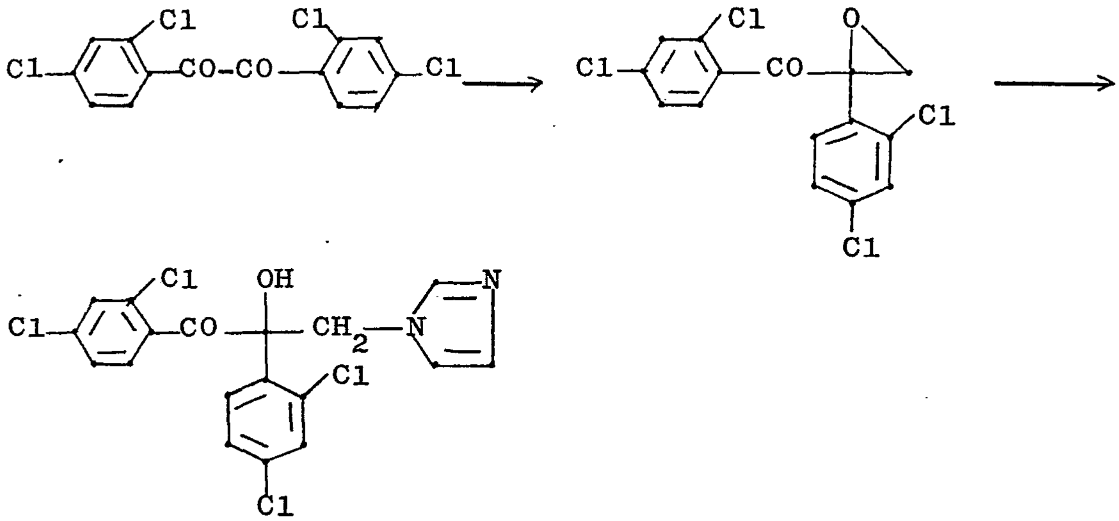

(1) An ethereal solution of diazomethane prepared from 3.1 g of N-nitrosomethylurea and aqueous potassium hydroxide is added to a solution of 3.4 g of 2,2',4,4'-tetrachlorobenzil in 50 ml of dioxane, and the mixture is stirred at room temperature for 16 hours. The reaction mixture is concentrated in vacuo to give 2-(2,4-dichlorobenzoyl)-2-(2,4-dichlorophenyl)oxirane as an oil.

(2) To a solution of the above product dissolved in 10.2 ml of dry dimethylformamide is added 4 g of 1H-imidazole, and the mixture is stirred at 70°C for 2,5 hours. Ice water is added to the reaction mixture, which is extracted with methylene chloride. The organic layer is washed with water, dried over sodium sulfate and then concentrated in vacuo. The residue is chromatographed on a column of silica gel and eluted with methylene chloride,

3 % methanol-methylene chloride, 5 % methanol-methylene chloride and 7 % methanol-methylene chloride, succesively. The eluates containing the objective compound are concentrated in vacuo. The residue is washed with ethyl acetate and recrystallized from dimethylformamide-ethyl acetate to give 1.45 g of 2',4'-dichloro-2-(2,4-dichlorophenyl)-2-hydroxy-3-(1H-imidazol-1-yl)propiophenone as crystals, m.p. 164 - 166°C. Yield 32.5 %.

The hydrochloride: m.p. 133 - 135°C (dec.).

Example 2 - 3

The reactions are performed as in Example 1, whereby the following products (Ia) are obtained.

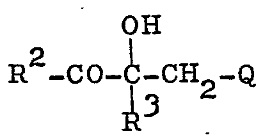

(Ia)

| Ex.No. | $R^2$ | $R^3$ | Q | mp(°C) |
|---|---|---|---|---|
| 2 | 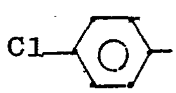 | 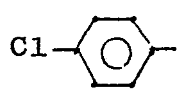 | 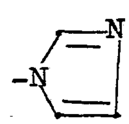 | 220-221 |
| 3 | 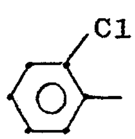 | 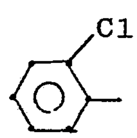 | 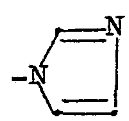 | 232-234 |

Example 4

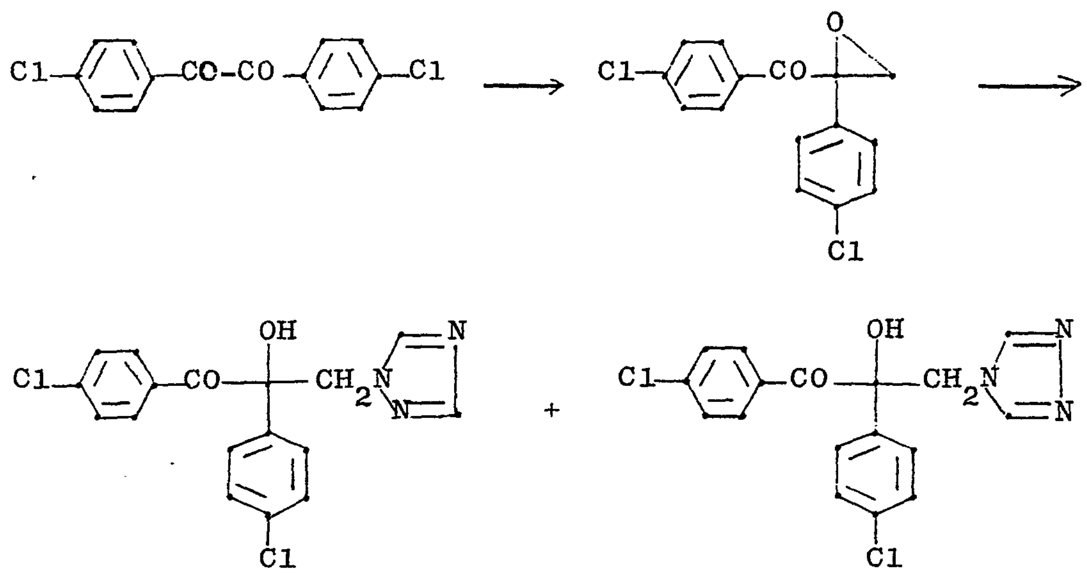

(1) An ethereal solution of diazomethane prepared in ether from 5.54 g of N-nitrosomethylurea and aqueous potassium hydroxide is added to a solution of 5 g of 4,4'-dichlorobenzil in 50 ml of dioxane, and the mixture is stirred at room temperature for 16 hours. The reaction mixture is concentrated in vacuo to give 2-(4-chlorobenzoyl)-2-(4-chlorophenyl)oxirane as an oil.

(2) A solution of the above product dissolved in 10 ml of dry dimethylformamide is added to a suspension of 1H-1,2,4-triazol-1-yl-sodium consisting of 1.9 g of 1H-1,2,4-triazole, 1.3 g of 50 % sodium hydride (dispersion in mineral oil) and 19 ml of dry dimethylformamide, and the mixture is stirred at 50°C for 20 minutes. The reaction mixture is concentrated in vacuo, and ice water is added to the residue. The mixture is extracted with methylene

chloride, and the organic layer is washed with water, dried over sodium sulfate and then concentrated in vacuo. The residue is chromatographed on a column of silica gel and eluted with methylene chloride, 2 % methanol-methylene chloride and 7 % methanol-methylene chloride in order. The eluate with 2 % methanol-methylene chloride is concentrated in vacuo. The residue is recrystallized from ether-isopropyl ether and ethyl acetate-isopropyl ether successively to give 1 g of 4'-chloro-2-(4-chlorophenyl)-2-hydroxy-3-(1H-1,2,4-triazol-1-yl)propiophenone as crystals, m.p. 102 - 104°C. Yield 12.8 %.

IR, $\nu_{max}^{Nujol}$ : 1670, 1650 $cm^{-1}$.

Then the eluate with 7 % methanol-methylene chloride is concentrated in vacuo. The residue is washed with ethyl acetate-ether and then recrystallized from methanol-ethyl acetate to give 420 mg of 4'-chloro-2-(4-chlorophenyl)-2-hydroxy-3-(4H-1,2,4-triazol-4-yl)propiophenone as crystals, m.p. 232 - 233°C. Yield 6.5 %.

IR, $\nu_{max}^{Nujol}$ : 1670 $cm^{-1}$.

Example 5 - 19

The following starting materials (VIII) may be treated in the same manner as in Example 4 to give the objective compounds (Ia) through the intermediates (II).

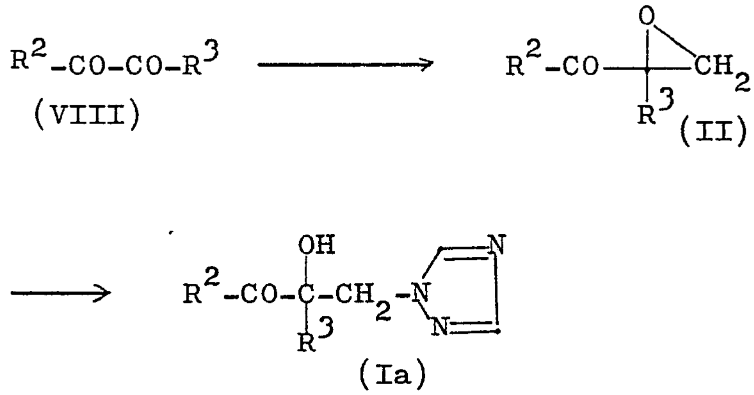

(VIII)

(II)

(Ia)

| Ex. No. | $R^2$ | $R^3$ | mp(°C) |
|---|---|---|---|
| 5 | Ph | Ph | 175 - 177*1 |
| 6 | 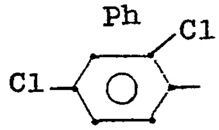 | 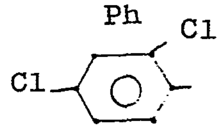 | 139 - 141*2 |
| 7 | 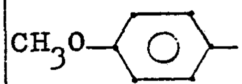 | 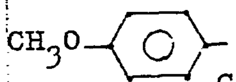 | 134 - 135 |
| 8 | 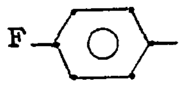 | 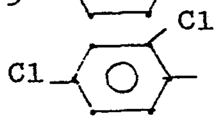 | 200 - 201*3 |
| 9 | 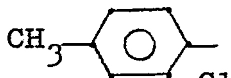 | 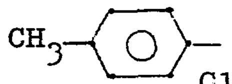 | 123 - 124 |
| 10 | 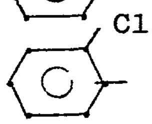 | 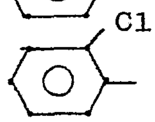 | 171 - 172 |
| 11 | 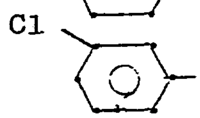 | 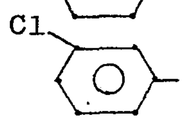 | 117 - 118 |
| 12 | 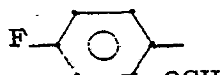 | 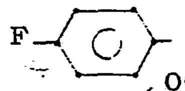 | 143 - 144*4 |
| 13 | 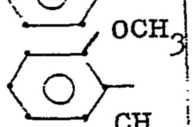 | 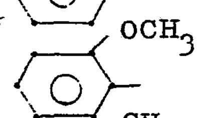 | 197 - 199 |
| 14 | 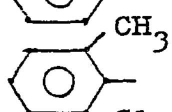 | 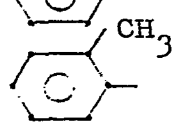 | 101 - 102 |
| 15 | 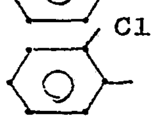 | Ph | 95 - 96 |
| 16 | 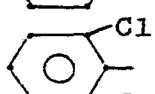 | 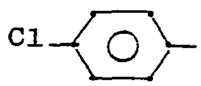 | 156 - 157 |
| 17 | 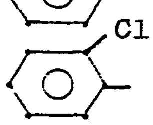 | 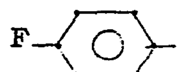 | 123 - 125 |
| 18 | 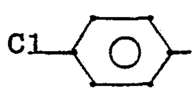 | 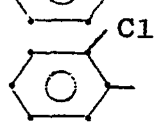 | 172 - 175 |
| 19 | 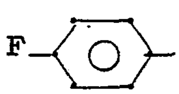 | 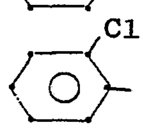 | 175 - 177 |

Note: Ph (Phenyl)

*1 4H-1,2,4-triazole isomer: m.p. 261 - 263°C
*2 1/5 ethyl acetate adduct
*3 4H-1,2,4-triazole isomer: m.p. 241 - 242°C
*4 4H-1,2,4-triazole isomer: m.p. 241 - 242°C

Example 20

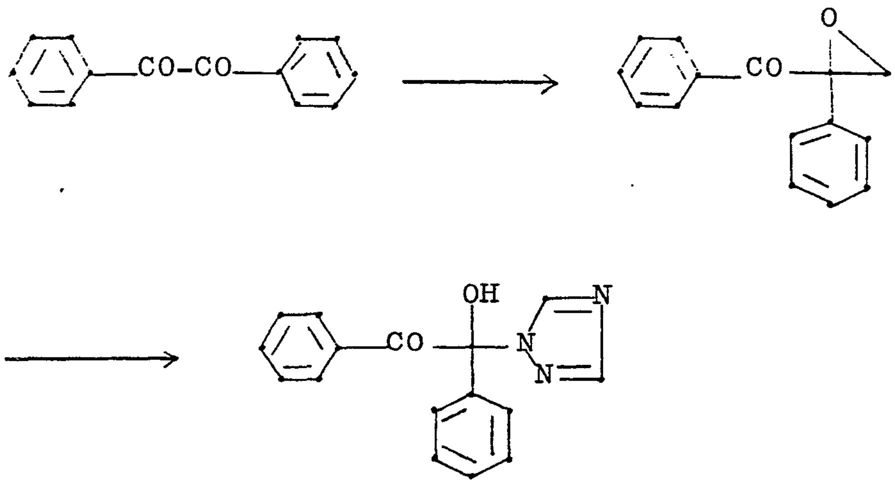

(1) Under nitrogen atmosphere, 1.43 g of 60 % sodium hydride dispersion in mineral oil is washed with dry petroleum ether, and 4.59 g of trimethyloxosulfonium chloride is added. Furthermore, 25 ml of dry dimethylsulfoxide is added to the reaction mixture under stirring at room temperature. The mixture is stirred for approximately 30 minutes until no hydrogen gas is evolved. To the reaction mixture is then slowly added 5 g of benzil at room temperature, and the stirring continued for 30 minutes. The reaction mixture is poured into ice water and extracted with ether. The ether layer is washed with water, dried over sodium sulfate and then concentrated. The residue (3.95 g) is chromatographed on a column of silica gel and eluted with benzene. The eluate containing the objective product is concentrated in vacuo to give 590 mg of 2-benzoyl-2-phenyloxirane as an oil.

Yield 11.1 %.

IR, $\nu_{max}^{film}$ : 1680 $cm^{-1}$.

[Einstert et al., Ber. 91, 2710 (1958)].

(2) The above product is allowed to react with sodium 1H-1,2,4-triazole in the same manner as in Example 4 (2) to give 2-hydroxy-2-phenyl-3-(1H-1,2,4-triazol-1-yl)-propiophenone as crystals, m.p. 175 - 177°C.

Example 21

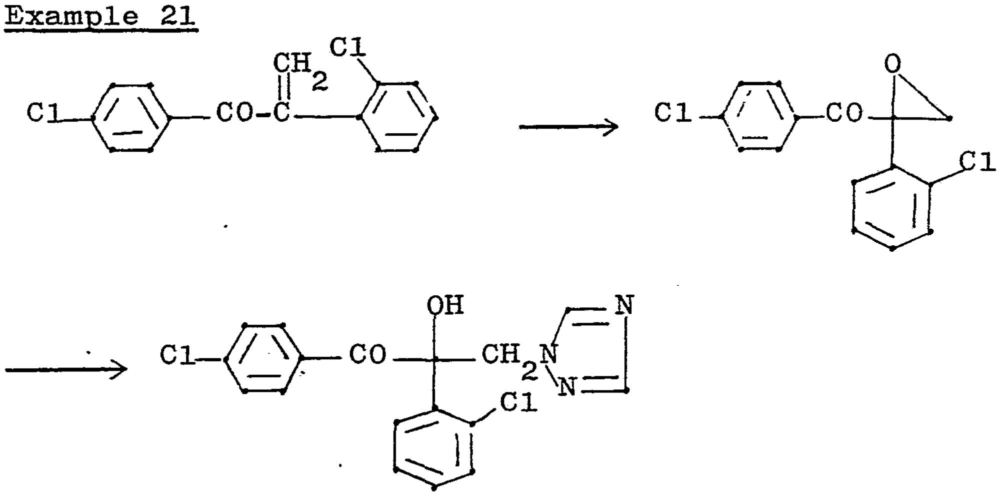

(1) To a solution of 150 ml of 1-(4-chlorobenzoyl)-2'-chlorostyrene dissolved in 8 ml of methanol are added 10 % aqueous sodium hydroxide and 1 ml of 30 % aqueous hydrogen peroxide, and the mixture is allowed to stand at room temperature for 14 hours. The reaction mixture is concentrated in vacuo and the residue is extracted with methylene chloride. The organic layer is washed with water, dried over sodium sulfate and then concentrated in vacuo. The residue is chromatographed on a column of silica gel, eluted with methylene chloride, and then the eluate containing the objective product is concentrated. The

resulting residue is washed with petroleum ether to give 25 mg of 2-(4-chlorobenzoyl)-2-(2-chlorophenyl)oxirane as crystals, m.p. 65 - 66°C.

IR, $\nu_{max}^{Nujol}$ : 1675 $cm^{-1}$.

(2) In the same manner as in Example 4 (2), 500 mg of the above product is allowed to react with 1H-1,2,4-triazol-1-yl-sodium to give 143 mg of 4'-chloro-2-hydroxy-2-(2-chlorophenyl)-3-(1H-1,2,4-triazol-1-yl)propiophenone as crystals, m.p. 172 - 175°C.

[Synthesis of the starting material]

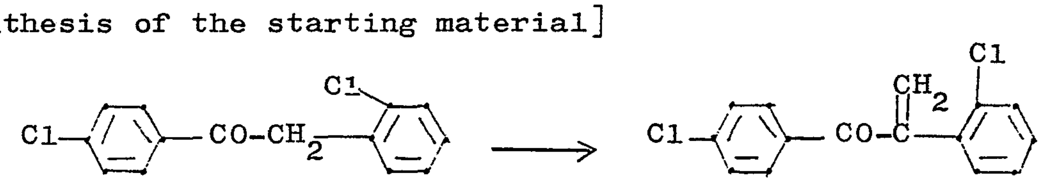

To 500 mg of 4'-chloro-2-(2-chlorophenyl)acetophenone are added 1 ml of acetic anhydride and 1 ml of N,N,N',N'-tetramethyldiaminomethane and the resulting solution is stirred at room temperature for 30 minutes. The reaction mixture is concentrated in vacuo, to which a small amount of methanol is added, and then concentrated again. The residue is washed with petroleum ether to give 280 mg of 1-(4-chlorobenzoyl)-2'-chlorostyrene as crystals, m.p. 120 - 122°C.

IR, $\nu_{max}^{Nujol}$ : 1655 $cm^{-1}$.

Example 22

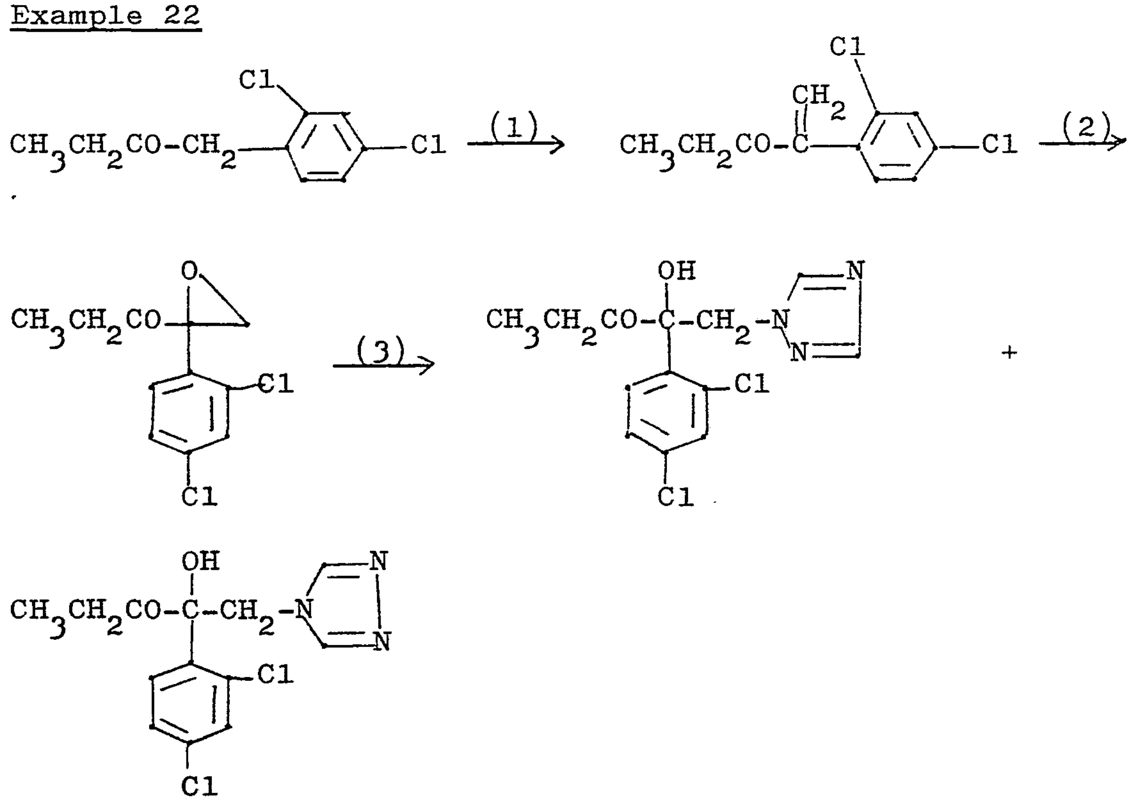


(1) To 1.34 g of acetic anhydride is added 1.9 g of 1-(2,4-dichlorophenyl)-2-butanone, and the mixture is dropwise mixed with 1.34 g of N,N,N',N'-tetramethyldiaminomethane with stirring to give a transparent solution with generation of heat. After 15 minutes, a small amount of methanol is added, and then the mixture is acidified with aqueous sodium bicarbonate and extracted with benzene. The extract is washed with water, dried over sodium sulfate and concentrated. The residue is chromatographed on a column of silica gel and eluted with methylene chloride. The eluate is evaporated to give 1.37 g of 4-(2,4-dichlorophenyl)-5-penten-3-one as an oil.

(2) To a solution of the product dissolved in 6.9 ml of acetone are added 0.28 ml of 10 % sodium hydroxide and 2.04 g of 30 % hydrogen peroxide, and the mixture is stirred for 15 minutes at room temperature. Chilled aqueous sodium hydrogensulfite is added to the mixture, which is eluted with methylene chloride. The eluate is washed with water, dried over sodium sulfate and evaporated. The residue is chromatographed on a column of silica gel and eluted with benzene. The eluates containing the objective compound are collected and evaporated to give 1.39 g of 2-(2,4-dichlorophenyl)-1,2-epoxy-3-pentanone as an oil.
IR $\nu_{max}^{film}$ 1710 $cm^{-1}$.

(3) To a solution of the product in 6.5 ml of dry dimethylformamide are added 550 mg of 1H-1,2,4-triazole, 50 % sodium hydride (oily dispersion), and the mixture is stirred with heating at 50°C for 3 hours. Ice water is added to the reaction mixture, which is extracted with a mixture of methanol and methylene chloride. The eluate is washed with water, dried over sodium sulfate and evaporated. The residue is chromatographed on a column of silica gel and eluted with 2 % methanol-methylene chloride; the eluates are collected and evaporated. The resulting residue is washed with isopropyl ether to give 1 g of the product. The product is recrystallized from methanol-ethyl acetate to give 800 mg of 2-(2,4-dichlorophenyl)-2-hydroxy-1-(1H-

1,2,4-triazol-1-yl)-3-pentanone as crystals, m.p. 205 - 208°C. Yield 48 %.

IR $\nu_{max}^{Nujol}$ 1705, 3100 $cm^{-1}$.

The column is further eluted with 7 % methanol-methylene chloride and the eluates are evaporated. The resulting residue is recrystallized from methanol-ethyl acetate to give 100 mg of 2-(2,4-dichlorophenyl)-2-hydroxy-1-(4H-1,2,4-triazol-4-yl)pentanone as crystals, m.p. 238 - 240°C. Yield 6 %.

<u>Example 23 - 58</u>

The reaction are performed as in Example 22, whereby the objective compounds (Ia) are obtained from the starting materials (VII) through the intermediates (VI) and (II).

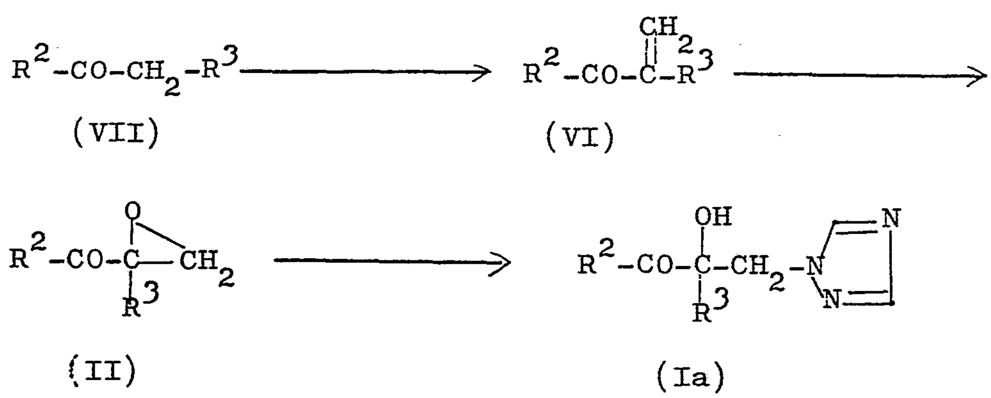

(VII) (VI)

(II) (Ia)

| Ex.No. | $R^2$ | $R^3$ | mp(°C) | Note |
|---|---|---|---|---|
| 23 | $CH_3CH_2CH_2CH_2$ | 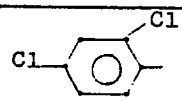 | 113 - 114 | |
| 24 | $CH_3$ | " | 181 - 183 | |
| 25 | $CH_3CH_2CH_2$ | " | 145 - 146 | |
| 26 | 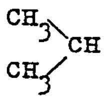 | " | 163.5 - 165 | |
| 27 | 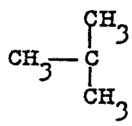 | " | 148.5 - 150 | |
| 28 | 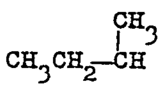 | " | 132 - 133 | |
| 29 | 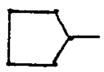 | " | 168.5 - 170.5 | |
| 30 | 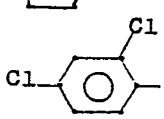 | $CH_3CH_2CH_2$ | 150 - 151(d) | oxalate |
| 31 | $CH_3CH_2CH_2$ | 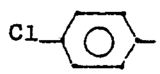 | 112 - 113 | |
| 32 | $CH_3CH_2CH_2$ | 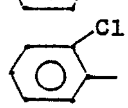 | 124 - 125 | |
| 33 | 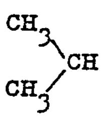 | 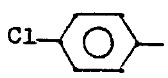 | 197(d) | oxalate |
| 34 | 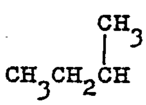 | " | 197(d) | oxalate |
| 35 | 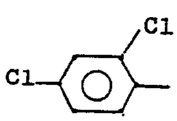 | " | 96.5 - 98<br>(101 - 103) | <br>(HCl salt) |
| 36 | $CH_3CH_2CH_2CH_2$ | " | 168 - 169 | oxalate |
| 37 | 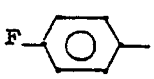 | " | 121 - 122 | |

| Ex.No. | $R^2$ | $R^3$ | mp(°C) | Note |
|---|---|---|---|---|
| 38 | 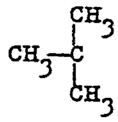 | 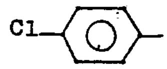 | 151 - 153 | |
| 39 | Ph | 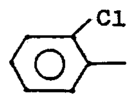 | 172 - 173 | |
| 40 | 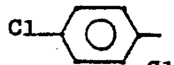 | " | 141 - 143 | |
| 41 | 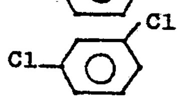 | " | 153 - 155 | |
| 42 | 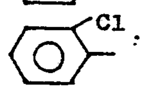 | 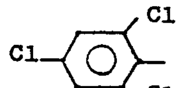 | 162 - 163 | |
| 43 | 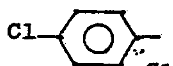 | 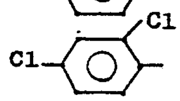 | 198 - 199 | |
| 44 | 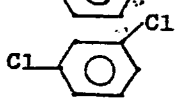 | 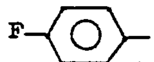 | 115 - 116 | |
| 45 | Ph | 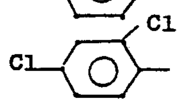 | 192 - 193 | |
| 46 | 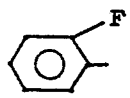 | " | 96 - 97 | DMF·$\frac{1}{5}H_2O$ |
| 47 | 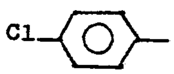 | 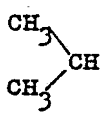 | 96.5- 97.5 | |
| 48 | " | $CH_3CH_2CH_2$ | 169 - 170(d) | oxalate |
| 49 | " | $CH_3CH_2CH_2CH_2CH_2$ | 169 - 171(d) | oxalate |
| 50 | " | 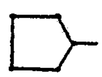 | 126 - 128(d) | oxalate |
| 51 | " | 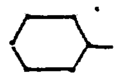 | 150 - 152(d) | oxalate |
| 52 | " | 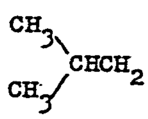 | 115 - 117(d) | oxalate |
| 53 | " | $CH_3CH_2CH_2CH_2$ | 75 - 75.5 | |

| Ex.No. | $R^2$ | $R^3$ | mp(°C) | Note |
|---|---|---|---|---|
| 54 | 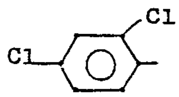 | $CH_3CH_2CH_2CH_2$ | 117 - 118 | oxalate |
| 55 | " | Ph | 117.5 - 118.5 | |
| 56 | " | $CH_3CH_2$ | 104 - 106 | |
| 57 | 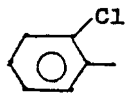 | $CH_3CH_2CH_2CH_2$ | 151 - 153(d) | oxalate |
| 58 | $CH_3CH_2CH_2CH_2$ | 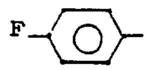 | 84 - 85 | |

Example 59

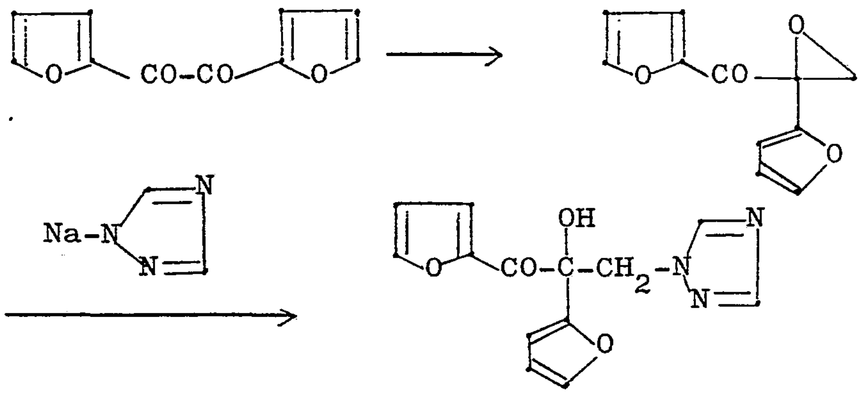

(1) To a solution of 1.9 g of furil in 19 ml of dimethylformamide is added a diazomethane ether solution prepared from the reaction of N-nitrosomethylurea (3.09 g) with 10 % potassium hydroxide, and the mixture is stirred for 30 minutes. Ice water is added to the mixture, which is extracted with ether. The extract is washed with water, dried over sodium sulfate and concentrated to give 1,2-bis(2-furyl)-(2,3-epoxy)-1-propanone as an oily residue.

(2) To a solution of the residue in 10 ml of dry dimethylformamide are added 1.03 g of 1H-1,2,4-triazole and 140 mg of 50 % sodium hydride dispersion (mineral oil), and the mixture is stirred at 50°C for 1.5 hours. Ice water is added to the mixture, which is extracted with methylene chloride. The extract is washed with water, dried over sodium sulfate and evaporated. The residue is chromatographed on a column of silica gel and eluted with methylene chloride and 2 % methanol-methylene chloride, and

the eluate is evaporated. The residue is crystallized from ether, filtered, and recrystallized from ethyl acetate-isopropyl ether to give 1,2-bis(2-furyl)-2-hydroxy-3-(1H-1,2,4-triazol-1-yl)-1-propanone as crystals, m.p. 154 - 155°C.

IR : $\nu_{max}^{Nujol}$ 1670 $cm^{-1}$.

Example 60

In the same manner as in Example 59, the objective compound 1,2-bis(2-pyridyl)-2-hydroxy-3-(1H-1,2,4-triazol-1-yl)-1-propanone can be provided from the starting material 2,2'-pyridil through the intermediate 1,2-bis-(2-pyridyl)-(2,3-epoxy)-1-propanone. m.p. 145.5 - 147°C.

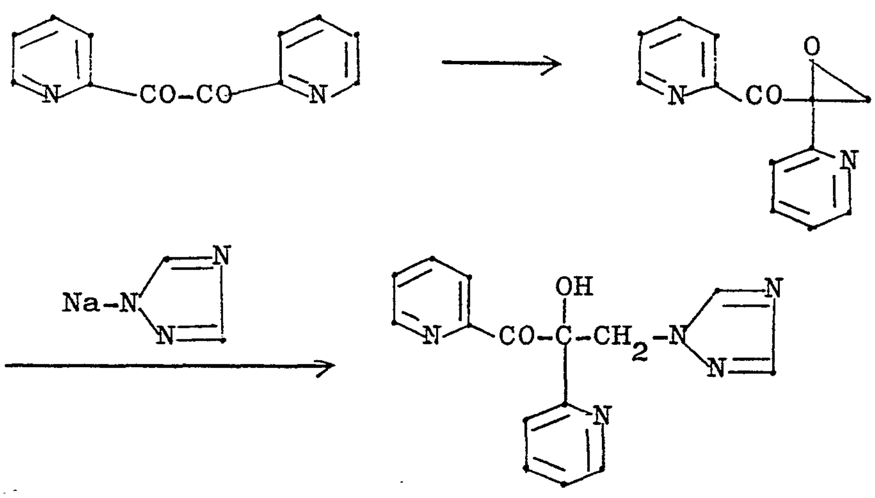

Example 61 - 62

The reaction is performed as in Example 22, whereby the following compounds are obtained.

Ex.61 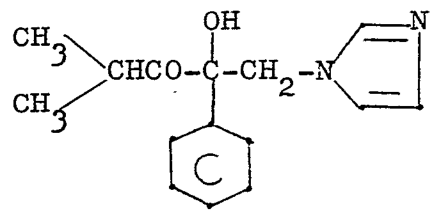 m.p. 184 - 186°C

Ex.62 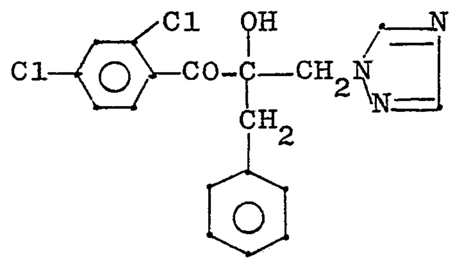 m.p. 110 - 111°C

Example 63

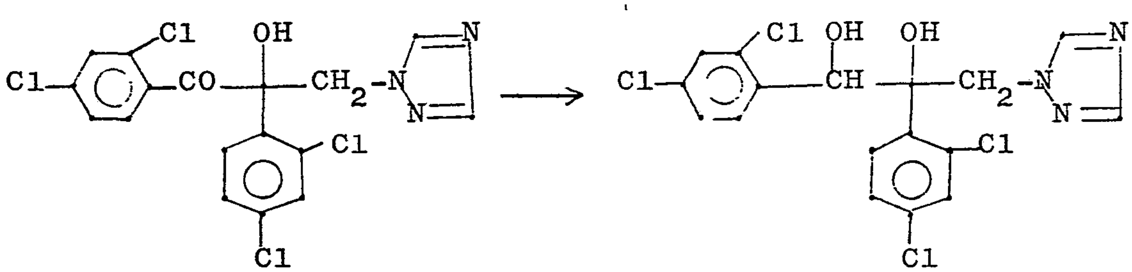

To a solution of 400 mg of 2',4'-dichloro-2-(2,4-dichlorophenyl)-2-hydroxy-3-(1H-1,2,4-triazol-1-yl)-propiophenone in 8 ml of ethanol is added 100 mg of sodium borohydride, and the mixture is heated on a water both for 5 minutes. The reaction mixture is concentrated in vacuo, and the residue is acidified with 6N hydrochloric acid, basified with aqueous sodium bicarbonate and extracted with methylene chloride. The organic layer is washed with water, dried over sodium sulfate and concentrated under reduced pressure. The resulting residue is chromatographed on a column of silica gel and eluted with 5 % methanol-methylene

chloride. The eluate is concentrated in vacuo, and the residual crystals are recrystallized from ethyl acetate-ether-isopropyl ether to give 150 ml of 1,2-bis(2,4-dichlorophenyl)-3-(1H-1,2,4-triazol-1-yl)propylene glyco 0.2 ethyl acetate adduct as crystals, m.p. 214 - 217°C. Yield 37.3 %. The hydrochloride, m.p. 106 - 110°C.

Example 64 - 83

The reactions are performed as in Example 63, wherei the following compounds (Ic) are obtained.

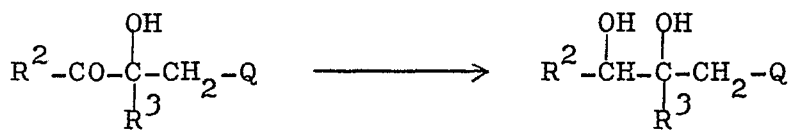

(Ia) (Ic)

| Ex.No. | $R^2$ | $R^3$ | Q | mp(°C) | Note |
|---|---|---|---|---|---|
| 64 | Ph | Ph | 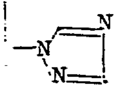 | 189 - 191 | 0.1 hydrate |
| 65 | 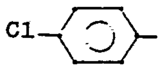 | 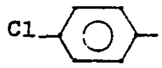 | " | 114 - 117 | 0.5 ethyl acetate adduct |
| 66 | 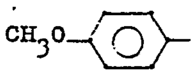 | 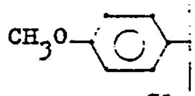 | " | 82.5 -83.5 | 0.5 ethyl acetate adduct |
| 67 | 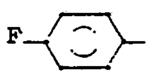 | 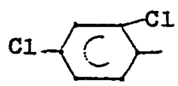 | " | 144 - 148 | 0.5 methanol adduct |
| 68 | 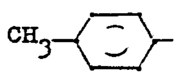 | 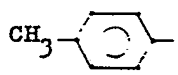 | " | 166 | |
| 69 | 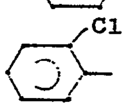 | 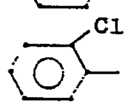 | " | 207.5 - 209.5 | |
| 70 | 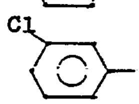 | 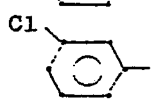 | " | 64 - 66 | 0.5 water, 0.5 isopropyl ether adduct |
| 71 | 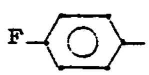 | 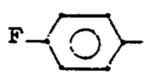 | " | 170 - 172 | |
| 72 | 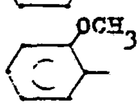 | 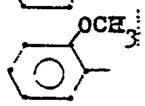 | " | 197 - 199 | 0.2 water, 0.5 ethyl acetate adduct |
| 73 | 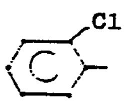 | Ph | " | 247 - 250 | |
| 74 | 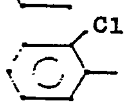 | 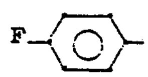 | " | 222 - 225 | |
| 75 | 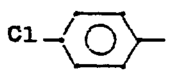 | 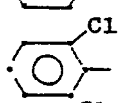 | " | 185 - 186 | |
| 76 | $CH_3CH_2CH_2$ | 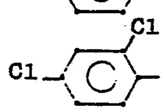 | " | 170 - 172 | |
| 77 | 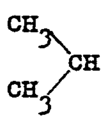 | 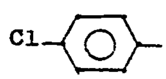 | " | 149 - 157 | |
| 78 | 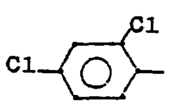 | 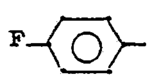 | 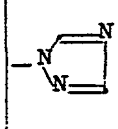 | 166 - 167.5 | |
| 79 | 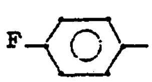 | 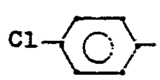 | " | 164 - 166 | |

| Ex.No. | $R^2$ | $R^3$ | Q | mp(°C) | Note |
|---|---|---|---|---|---|
| 80 | $CH_3CH_2CH_2$ | 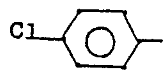 | 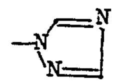 | 96 - 97 | |
| 81 | 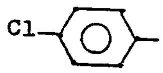 | 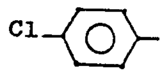 | 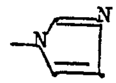 | 114 - 117 | 0.5 ethyl acetate adduct |
| 82 | 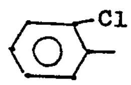 | 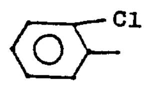 | " | 250 - 251 | |
| 83 | 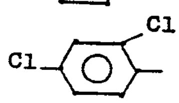 | 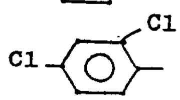 | " | 220 - 230 | |

Example 84

To a solution of 600 mg of 2-(4-chlorophenyl)-2-hydroxy-4-methyl-1-(1H-1,2,4-triazol-1-yl)pentan-3-one in 6 ml of dimethylformamide is added 147 mg of 50 % mineral oil dispersion of sodium hydride, and the mixture is stirred for 10 minutes under ice cooling. Then 289 mg of methyl chlorocarbonate is added to the mixture, which is stirred at room temperature for 30 minutes. The reaction mixture is mixed with ice water and shaken with ether. The organic layer is washed with water, dried over anhydrous sodium sulfate and concentrated in vacuo. The residue is chromatographed on a column of silica gel, which is eluted with methylene chloride and then 2 % methanol-methylene chloride. The eluate is concentrated in vacuo and the residue is crystallized from petroleum ether and recrystallized from isopropyl ether-petroleum ether to give 375 mg of 2-(4-chlorophenyl)-2-methoxycarbonyloxy-4-methyl-1-(1H-1,2,4-triazol-1-yl)pentan-3-one as crystals melting at 98 to 99°C.

IR : $\nu_{max}^{Nujol}$ 1720, 1750 $cm^{-1}$.

Example 85

To a solution of 5.0 g of 2-(4-chlorophenyl)-2-hydroxy-4-methyl-1-(1H-1,2,4-triazol-1-yl)pentan-3-one in 50 ml of methylene chloride are added 830 mg of 4-dimethylaminopyridine, 6.26 g of acetic anhydride and 5.15 g of triethylamine,

and the resultant mixture is refluxed for 3.5 hours. Ice water is added to the reaction mixture, which is shaken with methylene chloride. The organic layer is washed with water, dried over anhydrous sodium sulfate and concentrate in vacuo. The residue is chromatographed on a column of silica gel, which is eluted with methylene chloride and then 1 % methanol-methylene chloride. The eluate is concentrated in vacuo and the residue is twice crystallize from ethyl acetate-isopropyl ether to give 3.25 g of 2-(4-chlorophenyl)-2-acetoxy-4-methyl-1-(1H-1,2,4-triazol-1-yl)pentan-3-one as crystals melting at 126 to 127°C.

IR : $\nu_{max}^{Nujol}$ 1740, 1715 $cm^{-1}$.

Example 86 - 93

The reaction is performed as in Example 85, whereby the following compounds (Ib) are obtained.

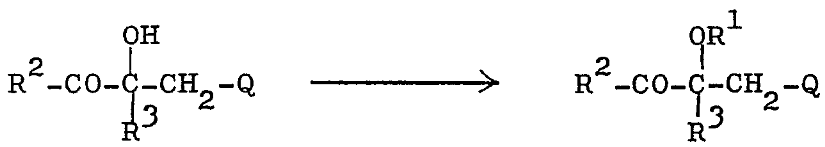

(Ia) (Ib)

| Ex.No. | $R^2$ | $R^3$ | $R^1$ | Q | mp(°C) |
|---|---|---|---|---|---|
| 86 | 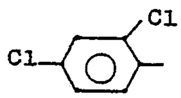 | 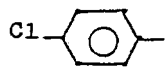 | 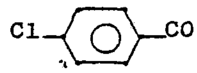 | 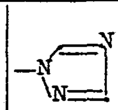 | 147 - 148 |
| 87 | " | " | $CH_3CO$ | " | 139 - 140 |
| 88 | " | " | $CH_3CH_2CO$ | " | 165 - 166 |
| 89 | " | " | PhCO | " | 181 - 182 |
| 90 | " | " | $CF_3CO$ | " | 202 - 203.5 |
| 91 | " | " | $COCH_2CH_2COOH$ | " | 176 - 177 |
| 92 | 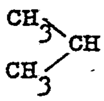 | 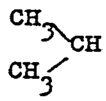 | $CH_3CO$ | " | 101 - 102* |
| 93 | 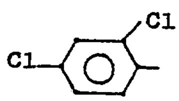 | 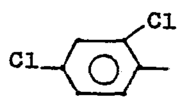 | $CH_3CO$ | 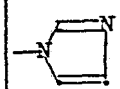 | 123 - 125 |

* 0.1 isopropyl ether adduct

Example 94 - 95

The reaction is performed as in Example 84, whereby the following compounds are prepared.

Ex.94 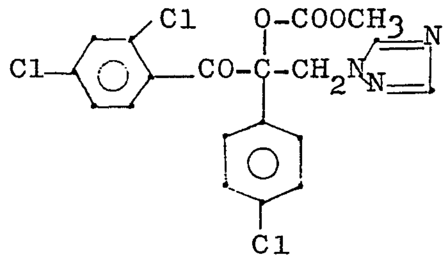 m.p. 142 - 144°C

Ex.95 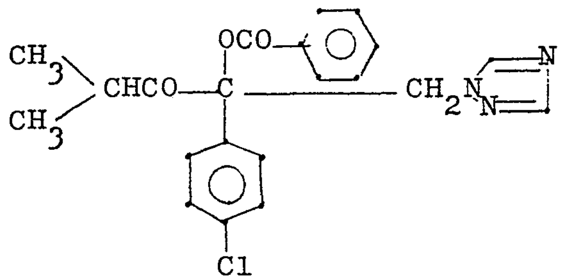 m.p. 151 - 152°C

Example 96

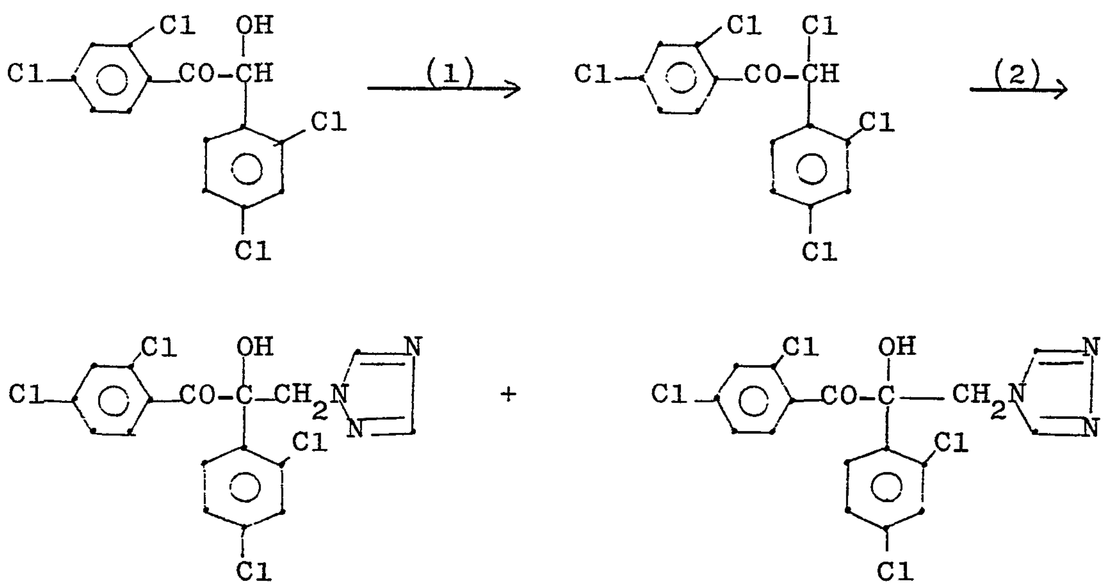


(1) A mixture of 2 g of 2,2',4,4'-tetrachlorobenzoin and 2.1 g of thionyl chloride is refluxed under heating

0117578

for 1.5 hours, and the reaction mixture is concentrated in vacuo. The residue is mixed with ice water and aqueous sodium bicarbonate and shaken with methylene chloride. The organic layer is washed with water, dried over anhydrous sodium sulfate and concentrated in vacuo. The residue is chromatographed on a column of silica gel, which is eluted with benzene-n-hexane (1/1, v/v). The eluate is concentrated in vacuo to give 2.2 g of 2-(2,4-dichlorophenyl)-2,2',4'-trichloroacetophenone as an oil.

IR $_{max}^{film}$ 1720 $cm^{-1}$.

(2) To a solution of 500 mg of 1H-1,2,4-triazole and 350 mg of 50 % mineral oil dispersion of sodium hydride in 4.4 ml of dry dimethylsulfoxide is added 270 mg of 80 % paraformaldehyde under ice cooling in nitrogen atmosphere. Then a solution of 2.2 g of the above product in 4.4 ml of dry dimethylsulfoxide is added to the mixture, which is stirred at room temperature for 40 hours. Ice water is added to the reaction mixture, which is extracted with ether. The organic layer is washed with water, dried over anhydrous sodium sulfate and concentrated in vacuo. The residue is chromatographed on a column of silica gel, which is eluted with methylene chloride, 1 % methanol-methylene chloride, 2 % methanol-methylene chloride and then 7 % methanol-methylene chloride. The eluate with 2 % methanol-methylene chloride is concentrated in vacuo. The

residue is twice crystallized from ethyl acetate-isopropyl ether to give 645 mg of 2',4'-dichloro-2-(2,4-dichlorophenyl)-2-hydroxy-3-(1H-1,2,4-triazol-1-yl)propiophenone as crystals melting at 166 to 167°C. The yield is overall 17 %.

IR : $\nu_{max}^{Nujol}$ 3150, 3080, 1699, 1580, 810 $cm^{-1}$.

Further the eluate with 7 % methanol-methylene chloride is concentrated in vacuo and the residue is washed with ethyl acetate-isopropyl ether to give 130 mg of 2',4'-dichloro-2-(2,4-dichlorophenyl)-2-hydroxy-3-(4H-1,2,4-triazol-4-yl)propiophenone as crystals melting at 134°C(dec). The yield is overall 4 %.

IR : $\nu_{max}^{Nujol}$ 3400 - 2500, 1695, 1578 $cm^{-1}$.

Example 97

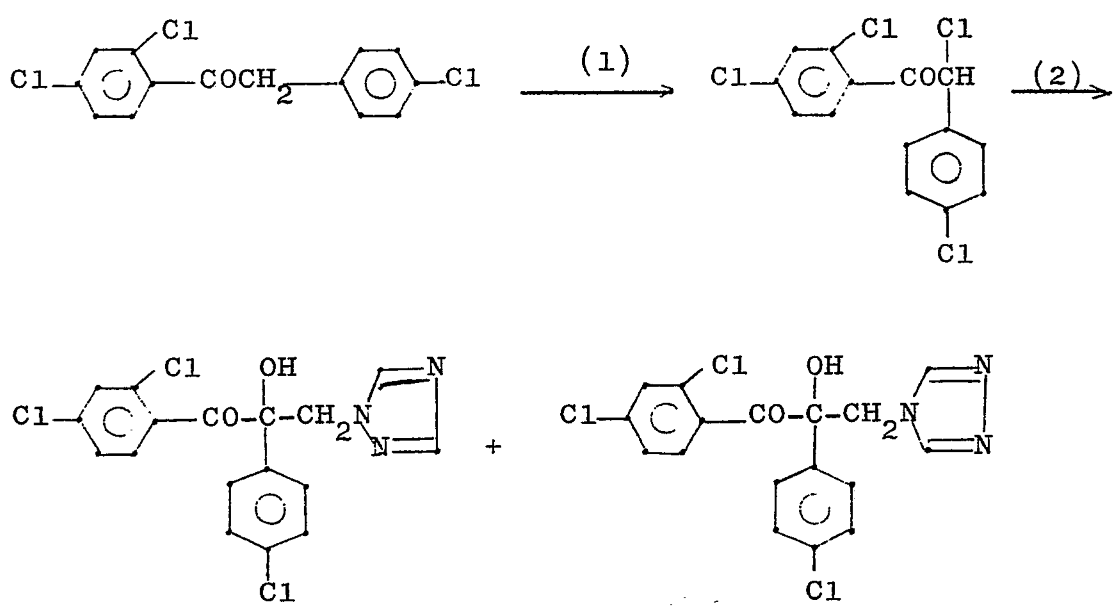


(1) A mixture of 2.9 g of 2-(4-chlorophenyl)-2',4'-dichloroacetophenone and 4 g of sulfuryl chloride is heated at 70°C for 1 hour. Ice water and aqueous sodium bicarbonate are added to the reaction mixture, which is extracted with methylene chloride. The organic layer is washed with water, dried over anhydrous sodium sulfate and concentrated in vacuo. The residue is chromatographed on a column of silica gel, which is eluted with benzene-n-hexane (1/1, v/v). The eluate is concentrated in vacuo to give 2.5 g of 2-(4-chlorophenyl)-2,2',4'-trichloroacetophenone as an oil.

IR : $\nu_{max}^{film}$ 1720, 1580 $cm^{-1}$.

(2) The above product is reacted as in Example 97 (2), whereby 690 mg of 2-(4-chlorophenyl)-2'4'-dichloro-2-hydroxy-3-(1H-1,2,4-triazol-1-yl)propiophenone is obtained as crystals melting at 92 to 95°C. The yield is overall 18 %.

IR : $\nu_{max}^{film}$ 3150, 1700, 1575 $cm^{-1}$.

Further 280 mg of 2-(4-chlorophenyl)-2',4'-dichloro-2-hydroxy-3-(4H-1,2,4-triazol-4-yl)propiophenone is obtained as crystals melting at 219 to 221°C.

IR : $\nu_{max}^{Nujol}$ 3170, 1685, 1570 $cm^{-1}$.

<u>Example 98</u>

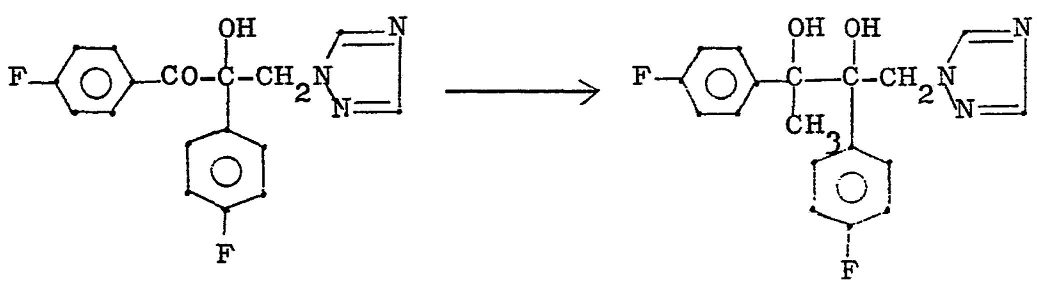

To a solution of 300 mg of 4'-fluoro-2-(4-fluorophenyl)-2-hydroxy-3-(1H-1,2,4-triazol-1-yl)propiophenone in 3 ml of dry tetrahydrofuran is added 4 ml of a solution of methylmagnesium bromide (4 mmol) in tetrahydrofuran, and the mixture is stirred at room temperature for 0.5 hours. The reaction mixture is concentrated in vacuo. The residue is acidified with 6N hydrochloric acid and neutralized with 10 % aqueous sodium hydroxide and shaken with methylene chloride. The organic layer is washed with water, dried over anhydrous sodium sulfate and chromatographed on a column of silica gel, which is eluted with benzene-ethyl acetate (1/1, v/v) and then with 10 % methanol-methyl acetate. The eluate with benzene-ethyl acetate (1/1, v/v) is concentrated in vacuo and the residue is dissolved in isopropyl ether and mixed with oxalic acid. The precipitating crystals are recrystallized from ethyl acetate-isopropylether to give 120 mg of 2,3-bis-(4-fluorophenyl)-1-(1H-1,2,4-triazol-1-yl)butane-2,3-diol monooxalate as crystals melting at 147 to 149°C.

Further the eluate with 10 % methanol-ethyl acetate is concentrated in vacuo and the residue is dissolved in isopropylether and mixed with oxalic acid. The precipitating crystals are recrystallized from ethyl acetate-isopropyl ether to give 60 mg of an isomer of said product as dioxalate. m.p. 96 to 98°C (dec).

Example 99

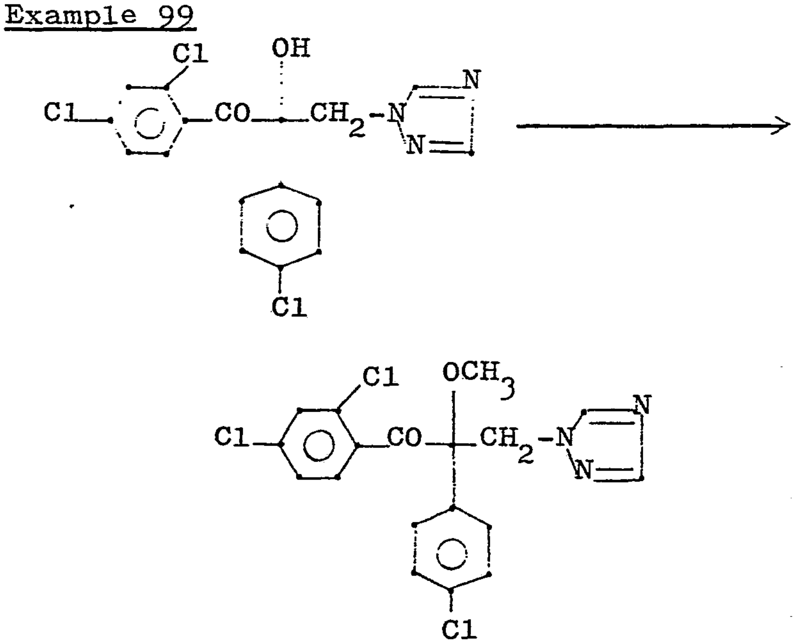

To a solution of 2 g of 2-(4-chlorophenyl)-2',4'-dichloro-2-hydroxy-3-(1H-1,2,4-triazol-1-yl)propiophenone in 10 ml of dry dimethylformamide is added 660 mg of 85 % potassium hydroxide, and the mixture is stirred at room temperature for 5 minutes. Then 1.5 g of methyl iodide is added to the mixture, which is stirred at room temperature for 30 minutes. Ice water is added to the reaction mixture, which is shaken with ether. The organic layer is washed with water, dried over anhydrous sodium sulfate and concentrated in vacuo. The residue is chromatographed on a column of silica gel, which is eluted with 1 % methanol-methylene chloride. The eluate is concentrated in vacuo, and the residue is washed with and crystallized from isopropyl ether-petroleum ether to give 550 mg of 2-(4-chlorophenyl)-2',4'-dichloro-2-methoxy-3-(1H-1,2,4-triazol-1-yl)-propiophenone as crystals melting at 121 to 122°C. The yield is 27 %.

IR., $\nu_{max}^{Nujol}$ 1690, 1578 $cm^{-1}$

Example 100

The reaction is performed as in Example 99, whereby the following compound is prepared.

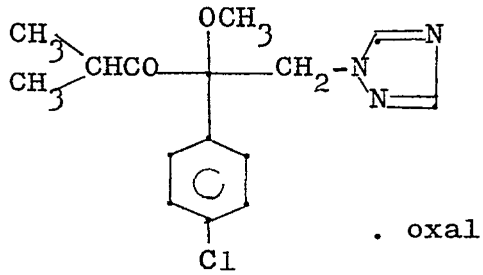

m.p. 141 - 142°C

. oxalate

Example 101

The product in Example 100 is reduced with sodium borohydride as in Example 63, whereby the following compound is prepared.

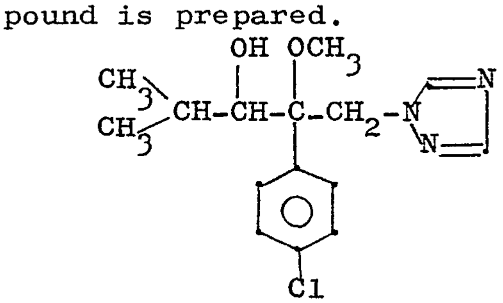

m.p. 126 - 132°C

Example 102

The product in Example 99 is reduced with sodium borohydride as in Example 63, whereby the following compound is prepared.

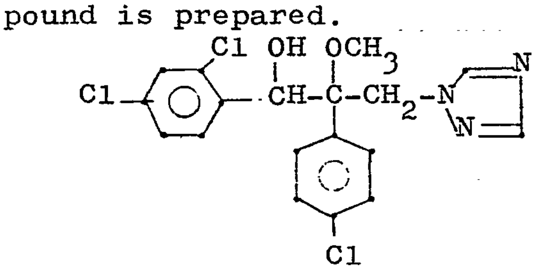

m.p.173 - 174°C
(isomer: m.p. 206-207°C)

The compounds used in the following Experiments are shown by the numbers which correspond to the numbers of the respective Examples.

Experiment 1

Minimum inhibitory concentration (MIC) of each test compound against Trichophyton asteroides in vitro antifungal test is shown below. Sabouraud's dextrose broth* was used as a culture medium.

* Totani et al., J. Med. Chem. 24, (12) 1492 (1981)

| Compound No. | MIC (μg/ml) |
|---|---|
| 1 | 0.1 |
| 6 | 0.1 |
| 7 | 12.5 |
| 9 | 1.56 |
| 10 | 0.1 |
| 11 | 3.13 |
| 23 | 0.1 |
| 25 | 0.1 |
| 29 | 0.1 |
| 32 | 0.8 |
| 34 | 0.1 |
| 63 | 0.1 |
| 67 | 0.1 |
| 69 | 0.1 |
| 71 | 1.56 |
| 80 | 1.6 |

Experiment 2

[Inhibition test against the formation of the pseudohyphae of Candida albicans]

To an Eagle's MEM medium (Nissan 2; Nissui Pharmaceutical Co., Ltd.) was added 20 % bovine serum, onto which yeast cells of Candida albicans KE-2 were inoculated so that the terminal inoculum size would amount to 1 x $10^6$ cells/ml. Then, a test compound was added to the medium by a two-fold dilution method, and cultivation was performed at 37°C for 18 hours. After termination of the cultivation, the cells of serial dilution on each medium were smeared and fixed on an object glass, then stained by the Giemsa's staining method, and the occurrence of the pseudohyphae was observed by microscopy. Minimum concentration for inhibiting the formation of the pseudohyphae was regarded as Inhibitory Concentration against Pseudohyphae Formation.

| Compound No. | Inhibitory Concentration (μg/ml) against Pseudohyphae Formation |
|---|---|
| 6 | 0.16 |
| 8 | 1.25 |
| 10 | 0.08 |
| 25 | 0.04 |
| 28 | 0.02 |
| 29 | 0.02 |
| 32 | 0.04 |
| 63 | 0.08 |
| 67 | 0.08 |
| 69 | 0.08 |
| 78 | 0.31 |

Experiment 3

[Therapeutic effect against experimental candidiasis in mice]

Candida albicans KE-2 was cultivated in a Sabouraud's dextrose agar at 28°C for 48 hours, and the resulting culture was suspended in the Sabouraud's dextrose broth. The cells ($5 \times 10^5$) were administered intravenously at the tail of female Jcl-ICR mice (4 weeks age, body weight 18 - 20 g). The test compound suspended in 2 % gum-arabic was orally administered at a dose of 50 mg/kg twice a day

for 5 days: on the first day, the test compound was administered twice i.e. immediately after the infection and 2 hours later; on the second day or later, 24 hours after the administration of the previous day respectively. An infected group to which no test compound was administered was regarded as a control group. Eight mice were employed per group in the control group and the test groups to which the test compound was administered. Therapeutic effect was evaluated on the survival rate at the 15th day since the day of infection; the result is shown below.

| Compound No. | Survival Rate (%) |
|---|---|
| 19 | 100 |
| 46 | 87.5 |
| 63 | 75 |
| 67 | 100 |
| control | 0 |

The compounds (I) or their acid addition salts show potent fungicidal activity against various phytopathogenic fungi and other soil born pathogens such as powdery mildew, downy mildew, sheath blight, damping-off or the like in vegetables, fruit plants, rice plant or other plants.

The compounds (I) or the salts thereof may be applied in a formulation suitable for agricultural fungicides such as emulsions, solutions,wettable powders, dusts, suspensions,

granules, aerosols, smokes, paste or the like. They may be applied singly or in combination with one or more of solid or liquid carriers, diluents and excipients. Representatives of the solid carriers, diluents and excipients are clay, talc, diatomaceous earth, silica, kaolin, bentonite, pumice and the like. Examplary carriers, diluents and excipients include water, methanol, ethanol, ethylene glycol, dimethylformamide, dimethylsulfoxide, acetone, methyl ethyl ketone, cellosolve, dioxane, diglyme and the like. When desired, there may be added appropriate adjuvants such as emulsifiers, dispersants, spreaders, surfactants, wetting agents, stabilizers, synergists or the like. Moreover, the compounds (I) or their salts may be used in combination with other agricultural chemicals such as other fungicides, germicides, insecticides, herbicides, repellents, miticides, nematocides, plant growth regulators or the like. Appropriate application rate of the compound (I) or its salt is in the range of about 10 - 500 ppm and about 2 - 200 g/10 are.

Experiment A

Control of powdery mildew in cucumber

Three ml of preparation containing a test compound at a concentration of 125 ppm was sprayed foliarly to the first leaf of young cucumber plant (cultivar: Matsukaze) which was grown in 9 cm pot in a glasshouse. Similar preparation

in 10 ml with the test compound at a concentration of 10 ppm was applied directly to the soil in another pot with cucumber. These plants were inoculated subsequently with conidia suspension of cucurbit powdery mildew (Sphaerotheca fuliginea) and kept in a glasshouse at the temperature of 25 ± 2 °C. Yet in another experiment cucumber in pot was inoculated first with conidia of Sphaerotheca fuliginea and after 2 days the test compound was sprayed foliarly as above to examine its suppressive ability against development of the disease. For all these cucumber plants were observed 10 days after each inoculation and degrees of mildew infection were given as follows:

0 : Disease sign was detected for nearly 100 % area in the inoculated leaf surface

3 : Disease sign was detected for about 50 % area

5 : Disease sign was detected for about 30 % area

7 : Disease sign was detected for about 20 % area

9 : Disease sign was slightly detected

10 : No disease sign was detected

The results are summarized in Table 1.

Table 1.

| Compound No. | Foliar spray (125 ppm) | | Soil application (10 ppm) |
|---|---|---|---|
| | Prevention | Suppression | Prevention |
| 1 | 10 | 10 | - |
| 12 | 10 | 10 | 10 |
| 24 | 10 | 10 | 10 |
| 25 | 10 | 10 | 10 |
| 26 | 10 | 10 | - |
| 27 | 10 | 10 | 10 |
| 30 | 10 | 10 | 10 |
| 31 | 10 | 10 | - |
| 32 | 10 | 10 | 10 |
| 39 | 10 | 10 | 3 |
| 46 | 10 | 10 | 5 |
| 71 | 10 | 10 | 10 |
| 76 | 10 | 10 | 10 |
| 90 | 10 | 10 | 10 |
| Untreated | 0 | 0 | 0 |

Experiment B

Control of cucumber downy mildew

The first leaf of young cucumber plant (cultivar : Matsukaze) grown in pot was sprayed with 3 ml of preparation containing a test compound at a concentration of 125 ppm. Five small droplets of suspension of sporangia of cucumber downy mildew (Pseudoperonospora cubensis) were applied to the leaf surface and the pot was kept at 22 ± 2°C under humid condition for 10 days. Then disease degree of downy mildew symptom developed was determined by the following method :

$$\text{Disease degree } (\%) = \frac{20a + 10b + 5c}{20(a+b+c+d)} \times 100$$

where,

a : Number of inoculations showing typical disease symptom

b : Number of inoculations at which chlorosis was developed

c : Number of inoculations at which chlorosis was slightly developed

d : Number of inoculations at which no disease symptoms were developed

$$\text{Control } (\%) = \frac{\text{Disease degree in untreated} - \text{Disease degree in treated}}{\text{Disease degree in untreated}} \times 100$$

These results are in Table 2.

Table 2.

| Compound No. | Appln. rate (ppm) | Disease degree (%) | Control (%) |
|---|---|---|---|
| 35 | 125 | 20 | 80 |
| 35* | 125 | 0 | 100 |
| 41 | 125 | 20 | 80 |
| 46 | 125 | 20 | 80 |
| Untreated | - | 100 | 0 |

* nitrate (m.p. 184°C dec.)

Experiment C

Control of sheath blight in rice

Rice (cultivar: Aichi-asahi) was grown in pots in a glasshouse for about 3 months and the plant in each pot was sprayed foliarly with 5 ml of preparation containing a test compound at a concentration of 500 ppm. Then the rice was inoculated at the stem near the soil with sheath blight fungus (Rhizoctonia solani) which was previously cultured in rice husks. The inoculated plant was placed in a humid (r.h. 97 %) chamber at 28°C for 5 days. Growth of mycelia grown upward along the rice sheath was measured to give rate of suppression:

$$\text{Rate of suppression (\%)} = \frac{\text{Growth of mycelia in untreated} - \text{Growth of mycelia in treated}}{\text{Growth of mycelia in untreated}} \times 100$$

Table 3 shows the results.

Table 3.

| Compound No. | Appln. rate (ppm) | Growth of mycelia (mm) | Suppression (%) |
|---|---|---|---|
| 22* | 500 | 21 | 90 |
| 25 | 500 | 20 | 90 |
| 26 | 500 | 9 | 95 |
| 27 | 500 | 14 | 93 |
| 33 | 500 | 18 | 91 |
| 77 | 500 | 23 | 89 |
| Untreated | - | 210 | 0 |

* 1H-1,2,4-triazol-1-yl isomer

Experiment D

Control of dumping-off in cucumber seedlings

Twenty seeds of cucumber (cultivar: Matsukaze) were sown in 150 g of sterilized soil in 9 cm pot. The soil surface was covered with 15 g of inoculum which contained a dumping-off pathogen, Rhizoctonia solani precultured in a mixture of wheat bran and sterilized soil. Then 30 ml of preparation with a test compound at a concentration of 500 ppm was applied to the soil and the pot was kept at 30 ± 2 °C in a humid chamber. Degree of dumping-off

was observed 14 days after the sowing and percent control was calculated as follows:

$$\text{Disease degree}(\%) = \frac{4a + 3b + 2c + d}{4(a + b + c + d + e)} \times 100$$

where, a : Number of seeds which did not germinate

b : Number of seedlings which showed typical dumping-off

c : Number of seedlings which showed wilting with various degrees

d : Number of seedlings whose hypocotyls or cotyledons showed some chlorosis

e : Number of seedlings which showed no symptoms

$$\text{Control}(\%) = \frac{\text{Disease degree in untreated} - \text{Disease degree in treated}}{\text{Disease degree in untreated}} \times 100$$

The results are in Table 4.

Table 4.

| Compound No. | Appln. rate (ppm) | Disease degree (%) | Control (%) |
|---|---|---|---|
| 8 | 500 | 5 | 95 |
| 10 | 500 | 8 | 92 |
| 22* | 500 | 10 | 90 |
| 26 | 500 | 20 | 80 |
| 33 | 500 | 10 | 90 |
| 35 | 500 | 10 | 90 |
| 45 | 500 | 0 | 100 |
| 46 | 500 | 0 | 100 |
| Untreated | - | 100 | 0 |

* 1H-1,2,4-triazol-1-yl isomer

Some illustrative formulations for agricultural fungicides are given below:

Formulation 1

A mixture of 5 parts by weight of Compound No. 12 (Compound prepared in Example 12), 85 parts by weight of clay and 10 parts by weight of talc is pulverized and formulated into a dust.

Formulation 2

A mixture of 50 parts by weight of Compound No. 24, 45 parts by weight of diatomaceous earth and 2.5 parts

by weight of calcium alkylbenzenesulfonate is pulverized, admixed and formulated into a wettable powder.

Formulation 3

A mixture of 30 parts by weight of Compound No. 30, 60 parts by weight of xylene and 10 parts by weight of polyoxyethylene alkyl aryl ether is stirred and formulated into an emulsion.

CLAIMS

1. A compound of the formula:

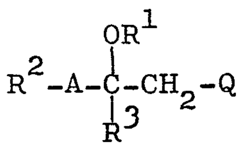

(wherein

A is C=O, CH(OH) or C($C_1$-$C_5$ alkyl)(OH),

Q is imidazolyl or triazolyl,

$R^1$ is hydrogen, $C_1$-$C_5$ alkyl or $C_1$-$C_8$ acyl and

$R^2$ and $R^3$ each is $C_1$-$C_5$ alkyl, $C_3$-$C_6$ cycloalkyl, $C_2$-$C_6$ alkenyl, benzyl optionally substituted by 1 to 3 halogens, 5- or 6-membered heterocycle containing N, O, or S or phenyl optionally substituted by 1 to 3 substituent selected from $C_1$-$C_3$ alkyl, $C_1$-$C_3$ alkoxy and halogen)

or its acid addition salt.

2. An oral antimycotic composition comprising an effective dosage of the compound claimed in claim 1 with one or more of pharmaceutically acceptable carriers, diluents and excipients.

3. An agricultural fungicidal composition comprising an effective amount of the compound claimed in claim 1 with one or more of pharmaceutically acceptable carriers, diluents and excipients.

4. A process for preparing the compound claimed in claim 1 which comprises reacting a compound of the formula:

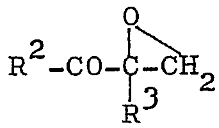 (II)

(wherein $R^2$ and $R^3$ each is as given in claim 1) with a compound of the formula:

Q-H (III)

(wherein Q is as given in claim 1) in the presence of an inorganic base in a solvent at temperature of from 20 to 150°C or reacting a compound of the formula:

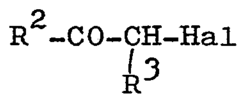 (IV)

(wherein Hal is halogen and $R^2$ and $R^3$ each is as given in claim 1) with paraformaldehyde and a compound of the formula:

Q-H (III)

(wherein Q is as given in claim 1) in the presence of an inorganic base in a solvent at temperature of from 0 to 100°C optionally followed by subjecting the resulting compound of the formula:

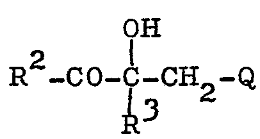 (Ia)

(wherein Q, $R^2$ and $R^3$ each is given in claim 1) to acylation, alkylation, reduction or Grignard reaction with a compound of the formula:

0117578

$R^4MgX$ (V)

(wherein X is halogen and $R^4$ is as given in claim 1) in accordance with methods already known.

5. A compound of the formula:

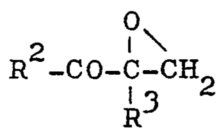

(wherein $R^2$ and $R^3$ each is $C_1$-$C_5$ alkyl, $C_3$-$C_6$ cycloalkyl, $C_2$-$C_6$ alkenyl, benzyl optionally substituted by 1 to 3 halogens, 5- or 6-membered heterocycle containing N, O or S or phenyl optionally substituted by 1 to 3 substituents selected from $C_1$-$C_3$ alkyl, $C_1$-$C_3$ alkoxy and halogen).